# EUROPÄISCHE PATENTSCHRIFT

(11) **EP 0 737 663 B1**
(45) Veröffentlichungstag und Bekanntmachung des Hinweises auf die Patenterteilung: **08.07.1998**
(21) Anmeldenummer: 96105406.1
(22) Anmeldetag: 04.04.1996
(51) Int. Cl.: C07C 45/69, C07C 67/347, C07C 253/30

(54) **Verfahren zur Herstellung von acetylenisch ungesättigten Verbindungen**
Process for the preparation of acetylenically unsaturated compounds
Procédé pour la préparation de composés acétyléniquement insaturés

(30) Priorität: 12.04.1995 DE 19513840
(43) Veröffentlichungstag der Anmeldung: 16.10.1996
(73) Patentinhaber: BASF Aktiengesellschaft, 67063 Ludwigshafen (DE)
(72) Erfinder: Rühl, Thomas Dr., 67227 Frankenthal (DE); Henkelmann, Jochem, Dr., 68165 Mannheim (DE); Heider, Marc, Dr., 67433 Neustadt (DE); Hofmann, Peter, Prof. Dr., 90475 Nürnberg (DE)

(56) Entgegenhaltungen:
- TETRAHEDRON LETTERS, Bd. 31, Nr. 48, 19.November 1990, Seiten 7063-7064, XP000168614 NIKISHIN G I ET AL: "NOVEL VINYL KETONE - ACETYLENE CROSS-COUPLING CATALYZED BY RHCL(PME3)3"

## Beschreibung

Die vorliegende Erfindung betrifft ein verbessertes Verfahren zur Herstellung von acetylenisch ungesättigten Verbindungen der allgemeinen Formel I

R¹-C≡C-CHR²-CH₂-X I,

in der die Substituenten folgende Bedeutung haben:
- R¹: Wasserstoff, gegebenenfalls substituierter Alkyl- oder Arylrest;
- R²: Wasserstoff oder C₁-C₄-Alkyl;
- X: CN; COOR³, wobei R³ für einen C₁-C₆-Alkyl- oder einen Benzylrest steht; COR⁴, wobei R⁴ für Wasserstoff oder einen C₁-C₄-Alkylrest steht,
durch Umsetzung eines Alkins der Formel II

R¹-C≡C-H II

mit einer α,β-ungesättigten Verbindung der Formel III

R²HC=CH-X III

in Gegenwart eines homogenen Rhodium-Phosphin-Katalysators.

Kovalev et al. beschreiben in Tetrahedron Letters 31 (1990) 7063 die Umsetzung von Alkinen wie Hexin und Phenylacetylen mit α,β-ungesättigten Ketonen wie Vinylmethylketon zu γ,δ-acetylenischen Ketonen. Als Katalysator dient Tris(trimethylphosphin)rhodiumchlorid. Befriedigende Umsätze sind nur nach langen Reaktionszeiten erzielbar. Die Reaktionszeiten, die im Bereich von mehreren Tagen liegen, sind für eine Umsetzung des beschriebenen Verfahrens in den technischen Maßstab prohibitiv.

Es bestand die Aufgabe, ein Verfahren bereitzustellen, das die Herstellung der Verfahrensprodukte I in hoher Ausbeute in kürzerer Reaktionszeit als nach dem Stand der Technik erlaubt.

Demgemäß wurde das eingangs beschriebene Verfahren zur Herstellung von acetylenisch ungesättigten Verbindungen der Formel I gefunden, das dadurch gekennzeichnet ist, daß man einen Rhodium-Katalysator mit einer zwei- oder dreizähnigen Phosphinverbindung verwendet.

Bei den erfindungsgemäß zu verwendenden Alkinen der Formel II handelt es sich um terminale Alkine. Neben Acetylen kommen alkylsubstituierte, vorzugsweise C₁-C₆-alkylsubstituierte Alkine in Betracht wie Propin, 1-Butin und 1-Hexin. Weiterhin kann ein arylsubstituiertes, vorzugsweise phenylsubstituiertes Alkin wie Phenylacetylen eingesetzt werden. Die Alkyl- und Arylreste können unter den Reaktionsbedingungen inerte Substituenten wie Halogen und Amino, vorzugsweise aber Hydroxy tragen. Beispielhaft für entsprechende Alkine sind Methylbutinol-1 und Propargylalkohol zu nennen. Besonders bevorzugte Alkine sind Acetylen, Hexin und Propargylalkohol.

Das Alkin der Formel II wird mit einer α,β-ungesättigten Verbindung der Formel III umgesetzt. Dabei kann es sich im Falle, daß der Substituent X für CN steht, um Nitrile wie Acrylnitril handeln.

Weiterhin kommen C₁-C₆-Alkylester, vorzugsweise Methyl- und Ethylester, sowie Benzylester von α,β-ungesättigten Säuren in Betracht, z.B. Acrylsäuremethylester, Acrylsäureethylester, Acrylsäure-tert.-butylester. Schließlich sind α,β-ungesättigte Aldehyde und Ketone wie Methylvinylketon, Crotonaldehyd, Cyclohexenon und 2(5H)-Furanon zu nennen.

Besonders bevorzugte Verbindungen der Formel III sind Methylvinylketon, Crotonaldehyd, Acrylsäureethylester und Acrylnitril.

Die Ausgangsverbindungen der Formeln II und III werden in der Regel in äquivalenten Verhältnissen umgesetzt.

Die Reaktion kann in einem Lösungsmittel erfolgen, dessen Menge im allgemeinen 5 bis 90 Gew.-%, bezogen auf die Reaktionsmischung, beträgt. Es kommen dazu Ketone wie Aceton, Ether wie Tetrahydrofuran und Methyl-tert.-butylether, aromatische Kohlenwasserstoffe wie Toluol und aprotische polare Lösungsmittel wie N-Methylpyrrolidon in Betracht.

Im allgemeinen erfolgt die Reaktion bei 20 bis 100°C, bevorzugt bei 30 bis 70°C. In der Regel wird bei atmosphärischem Druck gearbeitet, bei Einsatz von Acetylen jedoch kann der Druck bis zu 20 bar betragen.

Als Katalysator dient ein homogener Rhodium-Phosphin-Komplex. Die Phosphine sind zwei- oder dreizähnig, d.h. die erfindungsgemäß zu verwendenden Liganden weisen zwei oder drei tertiäre dreiwertige Phosphoratome, die bevorzugt nichtaromatische Reste tragen, auf. Bevorzugt tragen die Phosphoratome C₁-C₄-Alkylreste wie Methyl, Ethyl, n-Propyl, iso-Propyl, n-Butyl und tert.-Butyl sowie Cyclohexyl. Vorzugsweise werden zweizähnige Phosphine verwendet, deren Phosphoratome durch eine C₁-C₃-Alkylenkette getrennt sind, wie 1,2-Bis-(dicyclohexylphosphino)-ethan, 1,2-Bis-(dicyclohexylphosphino)-methan, 1,2-Bis-(dimethylphosphino)-ethan, 1,2-Bis-(dimethylphosphino)-methan, 1,2-Bis-(di-tert.-butylphosphino)-methan oder 1,2-Bis-(di-isopropylphosphino)-propan.

Solche mehrzähnigen Phosphine sind im Handel erhältlich oder nach literaturbekannten Methoden herstellbar.

Der Rhodium-Phosphin-Komplex wird vorzugsweise in situ hergestellt. Dazu wird ein zwei- oder dreizähniges Phosphin bevorzugt äquimolar oder mit einem bis zu 20 mol-%igen Phosphinüberschuß mit einer geeigneten Rhodiumverbindung umgesetzt, aus der sich unter den Reaktionsbedingungen ein Rhodium-Phosphin-Komplex bildet. Als Rhodiumverbindungen kommen Cyclooctadienylrhodiumchlorid, Rhodiumacetylacetonat und Rhodiumchlorid in Betracht.

Im allgemeinen werden Rhodiummengen von 0,01 bis 0,1 mol-%, bezogen auf das Alkin der Formel II, gewählt. Die entsprechenden Mengen kann der Fachmann durch Vorversuche leicht ermitteln.

Die Ausgangsverbindungen der Formel II und III sowie der Rhodiumphosphinkomplex oder seine Vorstufen (d.h. ein Phosphin und ein unter den Reaktionsbedingungen zum Phosphinkomplex reagierende Rhodiumverbindung) und gegebenenfalls ein Lösungsmittel können in einem Reaktor, z.B. einem Rührkessel oder bei Verwendung von Acetylen einem Druckkessel, vermischt werden, wobei vorteilhaft das Alkin als letzte Komponente zugegeben wird.

Die Reaktion kann sowohl kontinuierlich wie auch diskontinuierlich durchgeführt werden.

Nach einer Reaktionszeit, die im allgemeinen 2 bis 24 Stunden beträgt, wird das erhaltene Reaktionsgemisch in an sich bekannter Weise auf die Verfahrensprodukte der Formel I aufgearbeitet. Das Reaktionsgemisch kann destillativ vom Lösungsmittel befreit werden und das Produkt kann dann vom Katalysator abgetrennt werden. Dieser kann - gegebenenfalls nach Regenerierung - in die Reaktion zurückgeführt werden.

Das erfindungsgemäße Verfahren erlaubt die Herstellung acetylenisch ungesättigter Verbindungen der Formel I in hoher Ausbeute bei kurzen Reaktionszeiten.

Die Verfahrensprodukte der Formel I dienen als Bausteine für Zusätze in Galvanikbädern und als Pharmavorprodukte.

### Beispiele

### Allgemeine Herstellvorschrift

Zu 1 mmol Cyclooctadienylrhodiumchlorid in 30 ml Aceton wurden 1,1, mmol eines Phosphinliganden gegeben und 30 Minuten gerührt.

Zu der Lösung gab man 0,1 bis 1 mol der ungesättigten Verbindung der Formel III und 0,1 bis 1 mol des Alkins der Formel II.

Nach 12 h Rühren bei 50°C wurde das Verfahrensprodukt destillativ isoliert.

Die nachfolgende Tabelle gibt Einzelheiten zur Reaktion sowie die erzielten Ausbeuten an.

| Ungesättigte Verbindung III (mol) | Alkinverbindung II (mol) | Phosphinligand | Ausbeute (%) |
|---|---|---|---|
| MVK (0,1) | Hexin (0,1) | dmpe | 96 |
| MVK (0,1) | Hexin (0,1) | dmpm | 92 |
| MVK (0,1) | Hexin (0,1) | dcype | 93 |
| MVK (0,1) | Hexin (0,1) | dtbpm | 90 |
| MVK (0,1) | Acetylen (0,1) | dmpe | 55 |
| MVK (1) | Propargylalkohol (1) | dmpe | 93 |
| MVK (1) | Methylbutinol (1) | dmpe | 92 |
| Crotonaldehyd (0,1) | Hexin (0,1) | dmpe | 72 |
| Crotonaldehyd (0,1) | Acetylen (0,1) | dmpe | 34 |
| MVK = Methylvinylketon dmpe= 1,2-Bis-(dimethylphosphino)-ethan dmpm= 1,2-Bis-(dimethylphosphino)-methan dcype= 1,2-Bis-(dicyclohexylphosphino)-ethan dtbpm= 1,2-Bis-(di-tert.-butylphosphino)-methan | | | |

## Patentansprüche

1. Verfahren zur Herstellung von acetylenisch ungesättigten Verbindungen der allgemeinen Formel I
R¹-C≡C-CHR²-CH₂-X I,
in der die Substituenten folgende Bedeutung haben:
R¹ Wasserstoff, gegebenenfalls substituierter Alkyl- oder Arylrest;
R² Wasserstoff oder C₁-C₄-Alkyl;
X CN; COOR³, wobei R³ für einen C₁-C₆-Alkyl- oder einen Benzylrest steht; C(O)R⁴, wobei R⁴ für Wasserstoff oder einen C₁-C₄-Alkylrest steht,
durch Umsetzung eines Alkins der Formel II
R¹-C≡C-H II
mit einer α,β-ungesättigten Verbindung der Formel III
R²HC=CH-X III
in Gegenwart eines homogenen Rhodium-Phosphin-Katalysators, dadurch gekennzeichnet, daß man einen Katalysator mit einer zwei- oder dreizähnigen Phosphinverbindung verwendet.

2. Verfahren nach Anspruch 1, dadurch gekennzeichnet, daß man als α,β-ungesättigte Verbindung der Formel III Vinylmethylketon, Crotonaldehyd oder einen Acrylsäure-C₁-C₆-alkylester verwendet.

3. Verfahren nach Anspruch 1 oder 2, dadurch gekennzeichnet, daß man als Alkin der Formel II Acetylen, Propargylalkohol oder Hexin verwendet.

4. Verfahren nach den Ansprüchen 1 bis 3, dadurch gekennzeichnet, daß man als zweizähnigen Phosphinliganden 1,2-Bis-(dicyclohexylphosphino)-ethan, 1,2-Bis-(dicyclohexylphosphino)-methan, 1,2-Bis-(dimethylphosphino)-ethan, 1,2-Bis-(dimethylphosphino)-methan, 1,2-Bis-(di-tert.-butylphosphino)-methan oder 1,2-Bis-(di-isopropylphosphino)-propan verwendet.

## Claims

1. A process for preparing acetylenic compounds of the general formula I
R¹-C≡C-CHR²-CH₂-X I
where the substituents have the following meanings:
R¹ hydrogen, unsubstituted or substituted alkyl or aryl;
R² hydrogen or C₁-C₄-alkyl;
X CN, COOR³ where R³ is C₁-C₆-alkyl or benzyl; C(O)R⁴ where R⁴ is hydrogen or C₁-C₄-alkyl,
by reacting an alkyne of the formula II
R¹-C≡C-H II
with an α,β-unsaturated compound of the formula III
R²HC=CH-X III
in the presence of a homogeneous rhodium-phosphine catalyst, wherein a catalyst with a bidentate or tridentate phosphine compound is used.

2. A process as claimed in claim 1, wherein vinyl methyl ketone, crotonaldehyde or a C₁-C₆-alkyl acrylate is used as α,β-unsaturated compound of the formula III.

3. A process as claimed in claim 1 or 2, wherein acetylene, propargyl alcohol or hexyne is used as alkyne of the formula II.

4. A process as claimed in any of claims 1 to 3, wherein 1,2-bis(dicyclohexylphosphino)ethane, 1,2-bis(dicyclohexylphosphino)methane, 1,2-bis(dimethylphosphino)ethane, 1,2-bis(dimethylphosphino)methane, 1,2-bis(di-tert-butylphosphino)methane or 1,2-bis(diisopropylphosphino)propane is used as bidentate phosphine ligand.

## Revendications

1. Procédé de préparation de composés à insaturation acétylénique de formule générale I
R¹-C≡C-CHR²-CH₂-X I,
dans laquelle les substituants ont la signification suivante :
R¹ représente un atome d'hydrogène, un groupement alkyle ou aryle éventuellement substitué ;
R² représente un atome d'hydrogène ou un groupement alkyle en C₁-C₄ ;
X représente un groupement CN; COOR³ où R³ est mis pour un groupement alkyle en C₁-C₆ ou benzyle; C(O)R⁴ où R⁴ est mis pour un atome d'hydrogène ou un reste alkyle en C₁-C₄,
par réaction d'un alcyne de formule II
R¹-C≡C-H II
avec un composé α,β-insaturé de formule III
R²HC=CH-X III
en présence d'un catalyseur homogène rhodium/phosphine, caractérisé en ce que l'on utilise un catalyseur ayant un composé phosphine bi- ou tridenté.

2. Procédé selon la revendication 1, caractérisé en ce que l'on utilise en tant que composé α,β-insaturé de formule III de la méthylvinylcétone, du crotonaldéhyde ou un acrylate d'alkyle en C₁-C₆.

3. Procédé selon la revendication 1 ou 2, caractérisé en ce qu'on utilise en tant qu'alcyne de formule II, de l'acétylène, de l'alcool propargylique ou de l'hexyne.

4. Procédé selon l'une quelconque des revendications 1 à 3, caractérisé en ce que l'on utilise en tant que ligands phosphines bidentés, du 1,2-bis(dicyclohexylphosphino)éthane. du 1,2-bis(dicyclohexylphosphino)méthane du 1,2-bis(diméthylphosphino)éthane, du 1,2-bis(diméthylphosphino)méthane, du 1,2-bis(ditert.-butylphosphino)méthane ou du 1,2-bis(diisopropylphosphino)propane.
